# EUROPEAN PATENT SPECIFICATION

(11) **EP 0 967 915 B1**
(45) Date of publication and mention of the grant of the patent: **27.10.2004**
(21) Application number: 98959090.6
(22) Date of filing: 21.12.1998
(51) Int. Cl.: A61B 5/055, A61B 5/06, A61B 19/00, G01R 33/28

(54) **METHOD AND SYSTEM FOR POSITIONING A MAGNETIC RESONANCE VISIBLE DEVICE**
VERFAHREN UND SYSTEM ZUM POSITIONEREN EINER DURCH MAGNETISCHE RESONANZ SICHTBAREN VORRICHTUNG
PROCEDE ET APPAREIL DE MISE EN PLACE D'UN DISPOSITIF VISIBLE PAR LA RESONANCE MAGNETIQUE

(30) Priority: 31.12.1997 US 2561
(43) Date of publication of application: 05.01.2000
(60) Divisional application: 04100633.9
(73) Proprietor: Koninklijke Philips Electronics N.V., 5621 BA Eindhoven (NL)
(72) Inventor: MARTIN, Alastair, J., NL-5656 AA Eindhoven (NL); VAN VAALS, Johannes, Jacobus, NL-5656 AA Eindhoven (NL)
(74) Representative: Schouten, Marcus Maria
(86) International application number: PCT/IB1998/002087
(87) International publication number: WO 1999/034732

(56) References cited:
- US-A- 5 531 742
- US-A- 5 647 361
- US-A- 5 762 064

## Description

The invention relates to a method of imaging of an invasive device by means of magnetic resonance imaging for the purpose of establishing an anticipated trajectory of said device in a body, the method comprising the steps of acquiring MR images of the interior of a part of the body and selection of a target in the body and an entry point in the part of the body from the collection MR images acquired. The invention also relates to an MR system comprising an MR apparatus and an MR-visible device, the MR apparatus comprising means for generating a steady magnetic field, means for generating RF pulses, means for generating magnetic field gradient pulses, means for receiving MR-signals and means for processing the signals measured so as to form an image, and a control unit for generating control signals for the means for generating RF-pulses, the means for generating magnetic field gradient pulses, and the means for receiving MR signals. In the context of this application an MR visible device is to be understood to mean a device comprising means to be imaged in magnetic resonance images acquired from the device.

Such a method is known from US 5,647,361. The known method is used for MR guided intervention. For biopsies and similar interventions an MR visible device, for example an MR visible invasive device is guided along the anticipated trajectory to a target without intersecting sensitive structures between the surface of the body and the target, such as major blood vessels or brain tissue having an essential functionality. In the known method the anticipated trajectory from the entry point to the target in a body is selected from a collection of pre-intervention images. To this end at least one pre-intervention image is acquired from the region comprising the target in the body. The target is determined from the at least one pre-intervention image and an entry point is determined. In a next step of the known method the MR visible device is introduced in the body at the selected entry point and guided to the target by MR imaging of the MR-visible device. It is a drawback of the known method that the pre-intervention images might not actually represent a tissue location at the time of an actual intervention and that the sensitive structures between the target and the surface could be intersected.

It is *inter alia* an object of the invention to reduce the risk that the sensitive structures are intersected, in particular to provide technical means to support an operator to reduce said risk. To this end, a method in accordance with the invention is characterised in that in order to establish the anticipated trajectory between the target and the entry point in the body the method comprises further steps of at an instant prior to a positioning of an MR-visible device acquiring entry MR-images of said MR-visible device and a first part of the body, which comprises, at least a part of the anticipated trajectory near the entry point. In this way information on the actual position of the MR visible device and the surrounding tissue in the first part of the body can be updated by real time MR imaging and the anticipated trajectory can be established by a medical decision on the basis of the entry-images in such a way that the anticipated trajectory is chosen so that it does not intersect the sensitive structures between the surface and the target, not even if physiological changes of the tissue have occurred in the first part between the instant of pre-intervention imaging and the instant of actual intervention.

A particular version of the method in accordance with the invention is characterised in that the first part of the body comprises a slab extending parallel to a surface of the body containing the entry point. In this way the entry point can be localised on the body at the instant just before the intervention point while taking in account information of a possible change of the tissue location during the actual intervention and the instant of acquisition of pre intervention images, so that the MR-visible device can be positioned at the entry point.

Another version of the method as claimed in the invention is characterised in that the method comprises the further steps of
- acquiring first entry angle MR images from a second part of the body, comprising the anticipated trajectory and a first area near the entry point,
   displaying a line in the first entry angle MR images which represents the anticipated trajectory in the first entry angle MR images, providing targeting means for positioning the MR visible device at the actual entry point such that the MR visible invasive device is aligned relative to the displayed line. These steps constitute technical means for supporting the operator in establishing an entry angle of the MR-visible device at the entry point. The second part of the body may be coincide with the first part of the body.

Another version of the method in accordance with the invention is characterised in that in order to establish an angle of entry at the actual entry point the method comprises the further steps of acquiring second entry angle images from a third part comprising the anticipated trajectory and a second area near the entry point, the third part intersecting the second part through the anticipated trajectory, and positioning the MR-visible device using the targeting means at the actual entry point such that the MR-visible device is imaged simultaneously in both the first and the second entry angle images. In this way the MR visible pen is visible in both the first and the second entry angle images when it is aligned with the anticipated trajectory. These steps also constitute technical means for supporting the operator in establishing an angle of entry at the actual entry point. For example, said second and third parts comprise two orthogonal slabs which both contain the anticipated trajectory.

The MR visible invasive device may be imaged in the body while keeping the MR visible invasive device aligned with the line in the first entry angle MR images. The MR-visible device can thus be guided to the target.

The MR visible invasive device may be imaged in the body while keeping the MR visible invasive device remains simultaneously imaged in both the first and second entry angle MR images. The MR visible invasive device is thus visible in both the first and the second entry angle images and can be guided by taking into account deviations in the two planes.

The invention also relates to an MR-system comprising an MR imaging device and an MR-visible invasive device as defined in Claim 7. This MR-system of the invention is enabled to perform the method of Claim 1 and to acquire the described advantages.
These and other, more detailed, aspects of the invention will now be described and illustrated with reference to the Figures.
Figure 1 shows an MR apparatus,
Figure 2 shows an image of a slab and an entry point of a head of a body,
Figure 3 shows a plan view of a MR visible device,
Figure 4 shows a cross section 40 of the MR visible device,
Figure 5 shows two orthogonal slabs and an intersecting line representing the anticipated trajectory,
Figure 6 shows an example of a targeting device,
Figure 7 shows a movable guide and a guide plate of the targeting device,
Figure 8 shows a base plate and a suspension of the targeting device, and
Figure 9 shows an example of an MR visible pen.

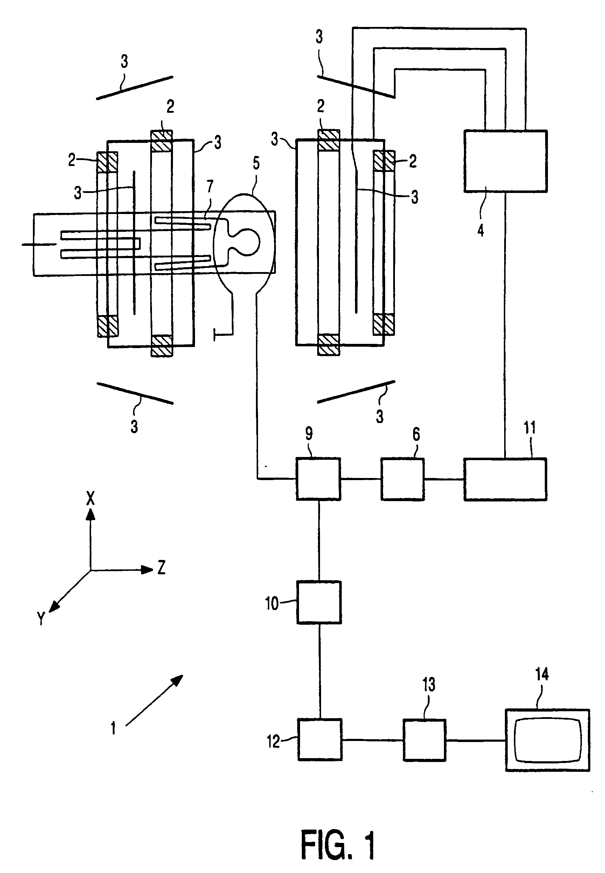

Fig. 1 shows an embodiment of an MR apparatus 1. The MR apparatus I comprises a first magnet system 2 for generating a steady magnetic field. The z-direction of the co-ordinate system shown corresponds to the direction of the steady magnetic field in the magnet system 2. The MR apparatus also comprises a second magnet system 3 for generating temporary magnetic fields directed in the z-direction and gradients in the x, y and z direction, respectively. It is to be noted that for ease of argument x, y and z are used for the frequency encoding direction, the phase encoding direction and the selection direction, respectively. These directions do not have to coincide with the main directions of the system. Furthermore, in this Application the temporary gradient fields having a gradient in the x-direction, the y-direction and the z-direction are referred to as a read gradient, a phase-encode gradient and a slice-selection gradient, respectively. Power supply means 4 feed the second magnet system 3. The magnet system 2 encloses an examination space which is large enough to accommodate a part of an object 7 to be examined, for example a part of a human body. An RF transmitter coil 5 serves to generate RF magnetic fields and is connected via a transmitter/receiver circuit 9 to an RF source and modulator 6. The RF transmitter coil 5 is arranged around the part of the body 7 in the examination space. The MR apparatus also comprises a receiver coil which is connected via the transmitter/receiver circuit 9 to a signal amplification and demodulation unit 10. The receiver coil and the RF transmitter coil 5 may be one and the same coil. A control unit 11 controls the modulator 6 and the power supply means 4 in order to generate MR imaging sequences comprising RF-pulses and temporary magnetic gradient fields. After excitation of nuclear spins in a part of the body placed within the examination space, the receiver coil 5 receives an MR signal. The phase and amplitude derived therefrom are sampled and further processed in the amplification and demodulation unit 10. An image reconstruction unit 12 processes the MR signals presented so as to form an image. The image is displayed via an image processing unit 13 for example on a monitor 14. The control unit 11 also controls the image reconstruction unit 12. Furthermore, the MR system comprises an MR visible device, for example an MR visible localizer, MR visible pen or an MR visible invasive device.

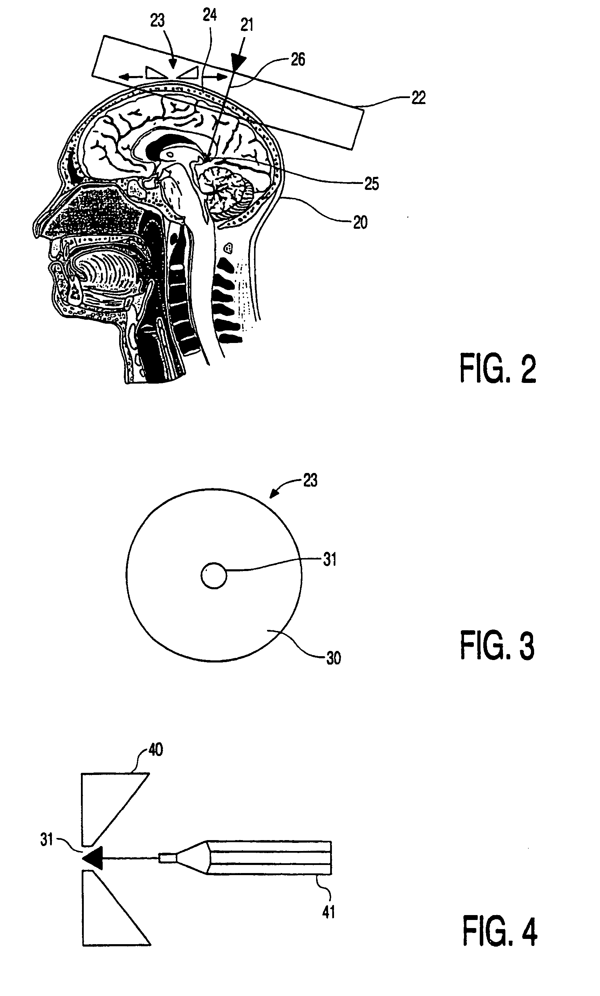

In a first version of the method as claimed in the invention a collection of pre-intervention MR images is acquired from a first part of the body, for example, the head, in order to identify a target lesion and sensitive tissue in the head in the vicinity of the lesion. MR imaging sequences employed for the acquisition of MR signals for the reconstruction of the pre-intervention MR images are, for example, Fast Field Echo (FFE) imaging sequences. Other feasible imaging sequences are, for example, Echo Planar Imaging (EPI) imaging sequences or Turbo Spin Echo (TSE) sequences. Fast Field Echo imaging sequences, EPI imaging sequences and TSE imaging sequences are all known from Magnetic Resonance Imaging, by M. T. Vlaardingerbroek et al, Springer Verlag, 1995. In a next step an operator selects an anticipated trajectory between a target in the body and an entry point in the part of the body from the collection of pre-intervention images. In order to establish the anticipated trajectory between the target and the entry point in the body at the instant before the intervention as claimed in the invention the body is positioned in the magnet room and further steps are performed to acquire entry MR-images of an MR-visible localizer and a first part of the head of the body comprising a part of the nominal trajectory between the target and the surface. Preferably, the first part comprises a first slab parallel to the surface. For the collection of the MR signals of the entry images, for example, the FFE imaging sequences, can be employed. The FFE imaging sequences are repeated to collect MR signals for the reconstruction of, for example from 2 to 5 images per second. The determination of the entry point is elucidated with reference to Figure 2.

Figure 2 shows an example of an entry MR image of the head of a body. The entry point 21 and the MR visible localizer 23 are also shown. The intersection of the slab and the skull is represented by a rectangle 22. In order to establish an actual entry point the reconstructed entry images 20 are displayed on the monitor 14. Preferably, the entry images are displayed on the monitor 14 in such a manner that the entry point is located at the centre of the image on the monitor. To identify the entry point in the entry images a marker can be superposed on the position of the entry point 21 represented in the displayed entry-images. Such a marker comprises, for example white or black cross-hairs.

In order to determine the entry point the MR-visible localizer 23 is moved across the surface 24 of the body. In this way the entry point 21 and the anticipated trajectory 26 between the entry point 21 and the target 25 can be determined just before the instant of the intervention from the entry-images on the monitor 14 while taking in account that the anticipated trajectory should reach the target 25 without intersecting sensitive structures within the skull. Sensitive structures within the skull are, inter alia, major brain vessels or brain tissue having an essential functionality. Additionally, a separate pre-intervention anatomical image or angiogram can be visualised as an overlay on the entry images so as to indicate the position of the major vessels.

The MR-visible localizer 23 comprises for example a ring which is composed of concentric rings or a circular symmetrical wedge-shape body containing an MR visible substance. Figures 3 and 4 show an example of an MR visible localizer.

Figure 3 is a plan view 30 of a MR visible localizer 23 which comprises a single ring 30 having a central orifice 31. Figure 4 shows an cross section 40 through the MR visible localizer 23. The ring contains for example an MR visible substance comprising a NiCl₂-solution in water which has a longitudinal relaxation time T₁ of 50 ms and a transversal relaxation time T₂ of 45 ms. The central orifice can be used for marking the skin with a pen 41 or a needle. Alternatively, the ring 30 could be provided with fixing means so as to fix it to the body's skin or skull. Another possibility is to attach the ring to an external arm fixed to the MR apparatus or the environment.

In order to improve the visualisation of the position of the MR-visible localizer 23, the MR imaging sequences used to collect the MR-signals for reconstructing the entry-images can be optimised so as to suppress or enhance different types of tissue. For example, Fast Field Echo imaging sequences comprising excitation RF pulses having a flip angle of 20 degrees can be employed. Another example of MR imaging sequences suitable for entry imaging are slice-selective projection MR angiogram sequences. such as a version of the FFE imaging sequence comprising excitation RF pulses having a flip angle of 90 degrees.

Furthermore, the image processor 13 can be arranged to analyse the image and automatically determine the position of the MR-visible localizer 23. This information can be used to reduce the field of view of the MR imaging sequences for the collection of MR-signals for the reconstruction of entry image, when the MR visible localizer 23 is closing in on the entry point 21, thereby increasing the spatial accuracy.

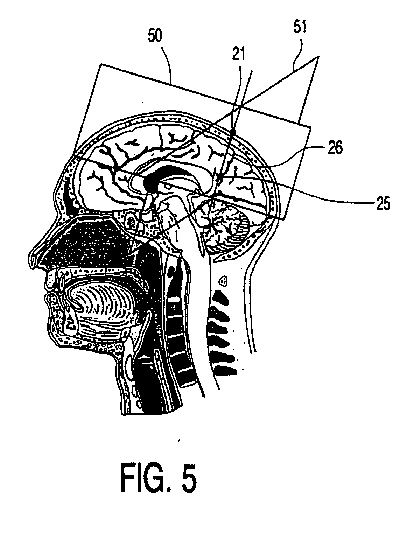

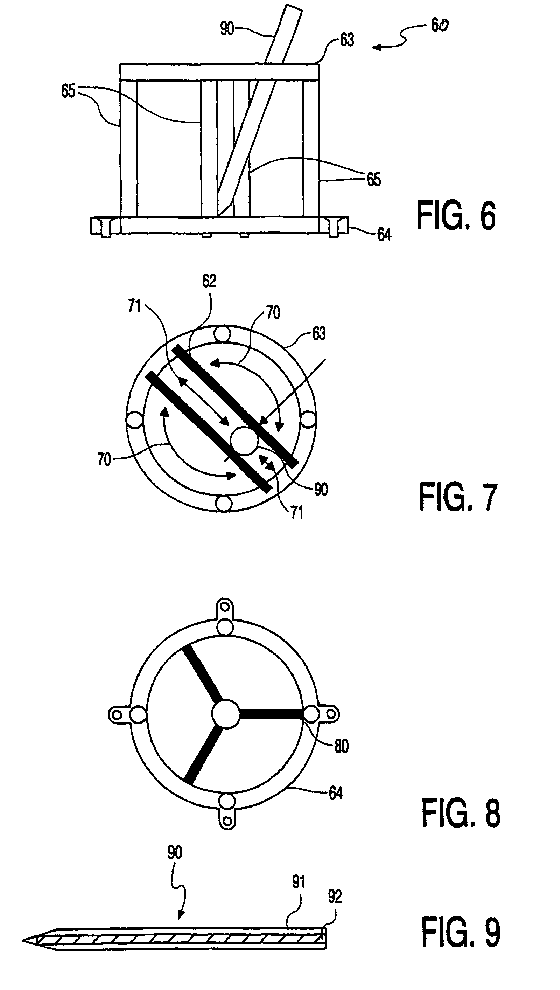

When the entry point has been established an entry angle can be established at which an MR visible pen can be aligned with respect to the anticipated trajectory. In order to establish the entry angle in a further version of the method as claimed in the invention first entry angle MR images are acquired from a second slab of the body which comprises the anticipated trajectory and a first area near the entry point. These further steps are elucidated with reference to Fig. 5. Figure 9 shows an example of an MR visible pen for use in the method as claimed in the invention.

Fig 5 shows an example of a second slab imaged in the first entry images. In Figure 5 the first slab is represented by a second rectangle 50. The second slab, represented by a third rectangle 51, comprises the anticipated trajectory 26 between the entry point 21 and the target 25. For example, the FFE sequences can also be used to collect the MR signals for the reconstruction of the first entry angle images. The image reconstruction unit 12 reconstructs the first entry angle images from the collected signals. The image processor 13 displays the first entry angle images on the monitor 14. The image processor 13 then plots a line 26 between the entry point 21 and the target 25 in the first entry angle MR images representing the anticipated trajectory in the first entry angle MR image, as shown in Figure 5, and an operator manipulates the MR-visible pen at the actual entry point 21 in such a manner that the MR visible pen becomes in line with the line 26. Such manipulation of the MR visible pen 90 can be performed by hand or by a targeting device. An example of a targeting device 60 will be described with reference to the Figures 6, 7 and 8.

In order to improve the entry angle of the MR visible pen 90 at the actual entry point the version of the method as claimed in the invention can be extended with a step for obtaining a second entry angle image from a third slab of the body comprising the anticipated trajectory and a second area near the entry point, the third slab intersecting the second slab through the anticipated trajectory. Figure 5 also shows an example of a third slab 51 of the second entry images. The second scan plane 51 is preferably chosen to be orthogonal to the second slab 50 and comprises the anticipated trajectory 26 as the intersection between the second and third slabs 50,51. For observation of the anticipated trajectory the image processor 13 displays, for example, both the first and second entry images on the monitor 14 and plots the line 26 representing the anticipated trajectory in the first and second entry angle images. In order to adjust the MR visible pen 90 to the correct entry angle the operator then manipulates the MR visible pen 90 at the actual entry point 21 in such a manner that the MR visible pen is simultaneously imaged in both the first and the second entry angle image. Thus the MR visible pen 90 is imaged in both the first and the second entry angle image only if it is aligned correctly. Once both the entry point and the entry angle have been set the MR visible pen 90 can be replaced by an MR visible invasive device, for example an MR visible needle.

In order to guide the MR visible needle to the target along the anticipated trajectory during the actual intervention first entry images are acquired from the second slab comprising the anticipated trajectory. The image processor 13 displays the first entry images on the monitor 14 and plots the line which represents the anticipated trajectory in the first entry images on the monitor, the operator introduces the MR-visible needle in the body while the MR-visible device remains aligned with the line in the first entry images. Alternatively, both first and second entry images can be acquired from the second and the third slab before and during the intervention. In order to display the anticipated trajectory the image processor 13 displays, for example, both the first and second entry images on the monitor 14 and the operator introduces the MR visible needle in the body while the MR visible needle remains imaged in both the first and second entry angle images simultaneously. The operator thus receives propartal information of the matching of the MR visible needle with the anticipated trajectory. The MR visible needle is completely visible in both the first and second entry angle images when it is perfectly matched with the anticipated trajectory and propartal visible in the first or second entry angle images, corresponding to a deviation from the anticipated trajectory.

In order to accelerate the procedure for adjustment of imaging parameters of the MR apparatus for acquiring the first entry images, the first and second entry angle images, the MR apparatus can be provided with a remote control device. The operator can then stay near the body so as to adjust the parameters.

Figure 6 shows an example of the targeting device 60, comprising a frame 61 and the MR visible pen 90 for use in the method in accordance with the invention. The frame 61 comprises a moveable guide 62 for positioning the MR visible pen 90, a guide plate 63 for co-operating with the moveable guide 62 and a base plate 64. The guide plate 63 and the base plate 64 are connected to one another by parallel rods 65. The frame is made of a non-metallic material, for example carbon fibre, ceramics or a synthetic material like such as Delrin.

Figure 7 shows the moveable guide 62 and the guide plate 63. Furthermore, first arrows 70 indicate a rotation of the guide and second arrows 71 indicate the translations of the MR visible pen 90. The moveable guide 62 is capable of rotating the MR visible pen 90 with respect to a first axis perpendicular to the base plate 64 of the targeting device and a second axis parallel to the base plate 64 and of translating the MR visible pen 90 in a plane parallel to the base plate 64. The targeting device 60 can also be provided with driving means (not shown) for rotating the MR visible pen 90 with respect to the first and second axes and for translating the MR visible pen 90. The driving means may be connected to a remote control receiver for receiving commands from a remote control device controlled by the operator.

Figure 8 shows the base plate and a suspension 80 for the fixation of a distal end of the MR visible needle at the entry point. The targeting device can be fixated over the actual entry point determined by connecting it to, for example the external arm associated with the MR apparatus, or by attaching the targeting device to the skull directly. Furthermore, the targeting device 60 can be provided with a slide sleeve (not shown in the figures ) for guiding the MR visible needle.

Figure 9 shows the MR visible pen 90 comprising of a housing 91 provided with a channel 92 centred along a length of the pen. The channel 92 contains an MR visible substance. The MR visible substance comprises, for example an NiCl₂ solution in water, having a longitudinal relaxation time T₁ of 50 ms and a transversal relaxation time T₂ of 45 ms.

## Claims

1. A method of imaging an invasive device (23,90) by means of magnetic resonance imaging for purposes of establishing an anticipated trajectory of said device in a body, the method comprising the steps of:
- acquiring MR images of a first part of the body;
- selecting the anticipated trajectory (26) between a target (25) in the body and an entry point (21) in the part of the body from the MR images acquired,
**characterized in that**
in order to establish the anticipated trajectory (26) between the target (25) and the entry point (21) in the body the method comprises further steps of
- at an instant prior to a positioning of said device acquiring entry MR-images (20) of said device (23,90) and a first part of the body which comprises at least a part of the selected anticipated trajectory (26) near the entry point (21).

2. A method as claimed in Claim 1, wherein the first part of the body comprises a slab (22) extending parallel to a surface (24) of the body containing the entry point (21).

3. A method as claimed in Claim 1 or 2, wherein the entry point (21) in the entry MR images (20) is represented by a marker in the entry MR-images (20).

4. A method as claimed in Claim 1, wherein the method also comprises the further steps of
- acquiring a first entry angle MR image (51) from a second part of the body comprising the anticipated trajectory (21) and a first area near the entry point (21),
- displaying a line in the first entry angle MR images (51) which represents the anticipated trajectory (26) in a first entry angle MR images and
- providing targeting means (60) for positioning the MR visible device (90) at the actual entry point (21) such that the MR visible device (90) is aligned relative to the line.

5. A method as claimed in Claim 4, wherein in order to establish an angle of entry at the actual entry point (21) the method also comprises the further steps of
- acquiring second entry angle images from a third part of the body, comprising the anticipated trajectory (26) and a second area near the entry point (21), the third part intersecting the second part at the anticipated trajectory (26), and
- positioning the MR visible device (90) using the targeting means (60) at the actual entry point (21) such that the MR visible device is imaged simultaneously in both the first and the second entry angle images.

6. A method as claimed in Claim 5, wherein said second and third parts comprising slabs of the body.

7. An MR system comprising an MR-device (1) and an MR-visible invasive device (90), the MR-device (1) comprising means for generating a steady magnetic field (2), means for generating RF pulses (5), means for generating magnetic filed gradient pulses (3), means for receiving MR-signals (9) and processing means (13) for processing the signals measured so as to form an image (20), and a control unit (11) for generating control signals for the means for generating RF-pulses (5), the means for generating magnetic filed gradient pulses (3), and the means for receiving MR signals (9), **characterized in that**
in order to establish an anticipated trajectory (26) between the target (25) and an actual entry point (21) in the body, the MR device is arranged to acquire entry MR-images (20) of the MR-visible device (90), MR-visible localizer (23) and a first part of a body which is to be placed in the MR device (1) and comprises a part of an anticipated trajectory (26) between the target (25) and the entry point (21), the processing means (13) being further arranged to analyze the MR-images (20) and to determine the position of the MR-visible localizer (23) automatically.

## Patentansprüche

1. Verfahren zum Abbilden einer invasiven Vorrichtung (23, 90) mittels Magnetresonanzbildgebung, um eine vorgesehene Bahn der genannten Vorrichtung in einem Körper festzulegen, wobei das Verfahren folgende Schritte umfasst:
- Erfassen von MR-Bildem eines ersten Teils des Körpers,
- Auswählen der vorgesehenen Bahn (26) zwischen einem Ziel (25) im Körper und einem Eintrittspunkt (21) im Teil des Körpers anhand der erfassten MR-Bilder,
**dadurch gekennzeichnet, dass**
das Verfahren zwecks Festlegung der vorgesehenen Bahn (26) zwischen dem Ziel (25) und dem Eintrittspunkt (21) im Körper weiterhin folgende Schritte umfasst:
- unmittelbar vor dem Positionieren einer genannten Vorrichtung Erfassen von Eintritts-MR-Bildem (20) der genannten Vorrichtung (23, 90) und eines ersten Teils des Körpers, der mindestens einen Teil der ausgewählten vorgesehenen Bahn (26) nahe des Eintrittspunkts (21) umfasst.

2. Verfahren nach Anspruch 1, wobei der erste Teil des Körpers eine Schicht (22) umfasst, die sich parallel zu einer den Eintrittspunkt (21) enthaltenden Oberfläche (24) des Körpers erstreckt.

3. Verfahren nach Anspruch 1 oder 2, wobei der Eintrittspunkt (21) in den Eintritts-MR-Bildern (20) durch eine Markierung in den Eintritts-MR-Bildern (20) dargestellt wird.

4. Verfahren nach Anspruch 1, wobei das Verfahren ferner folgende Schritte umfasst:
- Erfassen eines ersten Eintrittswinkel-MR-Bildes (51) von einem zweiten Teil des Körpers, umfassend die vorgesehene Bahn (21) und einen ersten Bereich nahe dem Eintrittspunkt (21),
- Anzeigen einer Linie in den ersten Eintrittswinkel-MR-Bildern (51), die die vorgesehene Bahn (26) in den ersten Eintrittswinkel-MR-Bildern darstellt, und
- Schaffen von Zielmitteln (60) zur Positionierung der MR-sichtbaren Vorrichtung (90) an dem tatsächlichen Eintrittspunkt (21) auf eine solche Weise, dass die MR-sichtbare Vorrichtung (90) relativ zu der Linie ausgerichtet wird.

5. Verfahren nach Anspruch 4, wobei das Verfahren zwecks Festlegung eines Eintrittswinkels am eigentlichen Eintrittspunkt (21) weiterhin folgende Schritte umfasst:
- Erfassen von zweiten Eintrittswinkelbildern von einem dritten Teil des Körpers, umfassend die vorgesehene Bahn (26) und einen zweiten Bereich nahe dem Eintrittspunkt (21), wobei der dritte Teil den zweiten Teil an der vorgesehenen Bahn (26) schneidet, und
- Positionieren der MR-sichtbaren Vorrichtung (90) unter Verwendung der Zielmittel (60) am eigentlichen Eintrittspunkt (21), so dass die MR-sichtbare Vorrichtung gleichzeitig sowohl in den ersten als auch in den zweiten Eintrittswinkelbildern abgebildet wird.

6. Verfahren nach Anspruch 5, wobei die genannten zweiten und dritten Teile Schichten des Körpers umfassen.

7. MR-System mit einem MR-Gerät (1) und einer MR-sichtbaren invasiven Vorrichtung (90), wobei das MR-Gerät (1) Mittel zur Erzeugung eines stationären Magnetfelds (2), Mittel zur Erzeugung von HF-Impulsen (5), Mittel zur Erzeugung von Magnetfeldgradientenimpulsen (3), Mittel zum Empfangen von MR-Signalen (9) und Verarbeitungsmittel (13) zur Verarbeitung der gemessenen Signale, um ein Bild (20) zu formen, umfasst sowie eine Steuereinheit (11), um Steuersignale für die Mittel zur Erzeugung von HF-Impulsen (5), die Mittel zur Erzeugung von Magnetfeldgradientpulsen (3) und die Mittel zum Empfangen von MR-Signalen (9) zu erzeugen, **dadurch gekennzeichnet, dass** das MR-Gerät zwecks Festlegung der vorgesehenen Bahn (26) zwischen dem Ziel (25) und einem tatsächlichen Eintrittspunkt (21) im Körper dafür eingerichtet ist, MR-Bilder (20) der MR-sichtbaren Vorrichtung (90), des MR-sichtbaren Lokalisierers (23) und eines ersten Teils des in das MR-Gerät (1) zu platzierenden Körpers zu erfassen und einen Teil einer vorgesehenen Bahn (26) zwischen dem Ziel (25) und dem Eintrittspunkt (21) umfasst, wobei die Verarbeitungsmittel (13) weiterhin dafür eingerichtet sind, die MR-Bilder (20) zu analysieren und die Lage des MR-sichtbaren Lokalisierers (23) automatisch zu bestimmen.

## Revendications

1. Procédé d'imagerie d'un dispositif invasif (23, 90) au moyen d'une imagerie par résonance magnétique dans le but d'établir une trajectoire anticipée dudit dispositif dans un corps, le procédé comprenant les étapes suivantes :
- l'acquisition d'images RM d'une première partie du corps;
- le choix de la trajectoire anticipée (26) entre une cible (25) dans le corps et un point d'entrée (21) dans la partie du corps à partir des images RM acquises,
**caractérisé en ce que**
pour établir la trajectoire anticipée (26) entre la cible (25) et le point d'entrée (21) dans le corps, le procédé comprend d'autres étapes :
- à un instant précédant le positionnement dudit dispositif, l'acquisition d'images RM d'entrée (20) dudit dispositif (23, 90) et une première partie du corps qui comprend au moins une partie de la trajectoire anticipée choisie (26) près du point d'entrée (21).

2. Procédé selon la revendication 1 dans lequel la première partie du corps comprend une plaque (22) s'étendant parallèlement à une surface (24) du corps contenant le point d'entrée (21).

3. Procédé selon la revendication 1 ou 2 dans lequel le point d'entrée (21) dans les images RM d'entrée (20) est représenté par un marqueur dans les images RM d'entrée (20).

4. Procédé selon la revendication 1 dans lequel le procédé comprend en outre les autres étapes suivantes :
- l'acquisition d'une image RM sous un premier angle d'entrée (51) d'une deuxième partie du corps comprenant la trajectoire anticipée (21) et une première zone près du point d'entrée (21),
- l'affichage d'une ligne dans les images RM sous le premier angle d'entrée (51), qui représente la trajectoire anticipée (26) dans les images RM sous le premier angle d'entrée, et
- la mise en oeuvre d'un moyen de ciblage (60) pour positionner le dispositif visible par RM (90) au point d'entrée réel (21) de sorte que le dispositif visible par RM (90) soit aligné par rapport à la ligne.

5. Procédé selon la revendication 4 dans lequel, pour établir un angle d'entrée au point d'entrée réel (21), le procédé comprend également les autres étapes suivantes :
- l'acquisition d'images sous le deuxième angle d'entrée d'une troisième partie du corps, comprenant la trajectoire anticipée (26) et une seconde zone près du point d'entrée (21), la troisième partie coupant la deuxième partie à la trajectoire anticipée (26), et
- le positionnement du dispositif visible par RM (90) en utilisant les moyens de ciblage (60) au point d'entrée réel (21) de sorte que le dispositif visible par RM forme une image simultanément dans les images sous le premier et le second angle d'entrée à la fois.

6. Procédé selon la revendication 5 dans lequel lesdites deuxième et troisième parties comprennent des plaques du corps.

7. Système RM comprenant un dispositif RM (1) et un dispositif invasif visible par RM (90), le dispositif RM (1) comprenant des moyens pour générer un champ magnétique stable (2), des moyens pour générer des impulsions RF (5), des moyens pour générer des impulsions de gradients de champs magnétiques (3), des moyens pour recevoir des signaux RM (9) et des moyens de traitement (13) pour traiter les signaux mesurés de manière à former une image (20), et une unité de commande (11) pour générer des signaux de commande pour les moyens de génération d'impulsions RF (5), les moyens de génération d'impulsions de gradients de champ magnétiques (3) et les moyens de réception de signaux RM (9), **caractérisé en ce que**
pour établir une trajectoire anticipée (26) entre la cible (25) et un point d'entrée réel (21) dans le corps, le dispositif RM est aménagé pour acquérir des images RM d'entrée (20) du dispositif visible par RM (90), du localisateur visible par RM (23) et d'une première partie d'un corps à placer dans le dispositif RM (1) et comprend une partie d'une trajectoire anticipée (26) entre la cible (25) et le point d'entrée (21), les moyens de traitement (13) étant en outre aménagés pour analyser les images RM (20) et déterminer la position du localisateur visible par RM (23) automatiquement.
